(19) Europäisches Patentamt
European Patent Office
Office européen des brevets

(11) Publication number: **0 311 365 A1**

# (12) EUROPEAN PATENT APPLICATION

(21) Application number: 88309260.3

(22) Date of filing: 05.10.88

(51) Int. Cl.⁴: **C07D 471/04 , A61K 31/435 , //(C07D471/04,221:00,205:00)**

Claims for the following Contracting States: ES + GR.

(30) Priority: 07.10.87 US 105762

(43) Date of publication of application: 12.04.89 Bulletin 89/15

(84) Designated Contracting States: AT BE CH DE ES FR GB GR IT LI LU NL SE

(71) Applicant: ELI LILLY AND COMPANY
Lilly Corporate Center
Indianapolis Indiana 46285(US)

(72) Inventor: Brennan, John
5514 Indian Cove Road
Indianapolis Indiana 46268(US)

(74) Representative: Hudson, Christopher Mark et al
Erl Wood Manor
Windlesham Surrey GU20 6PH(GB)

## (54) Monohydrate and solvates of a new beta-lactam antibiotic.

(57) The crystalline monohydrate, mono(N,N′-dimethylformamide) and bis(N,N′-dimethylformamide) solvates of 7β-[2′-(R)-2′-(p-hydroxyphenyl-2′-aminoacetamido]-3-chloro-3-(1-carbadethiacephem)-4-carboxylic acid ("LY213735") are novel compounds with superior physical characteristics. Also described are pharmaceutical formulations of crystalline LY213735 monohydrate.

EP 0 311 365 A1

# MONOHYDRATE AND SOLVATES OF A NEW β-LACTAM ANTIBIOTIC

This invention relates to a novel monohydrate and solvates of a β-lactam antibiotic, more particularly to a novel crystalline monohydrate and the mono- and bis-(N,N′-dimethylformamide)solvates of a 1-carbacephalosporin.

The β-lactam antibiotic of the Formula I

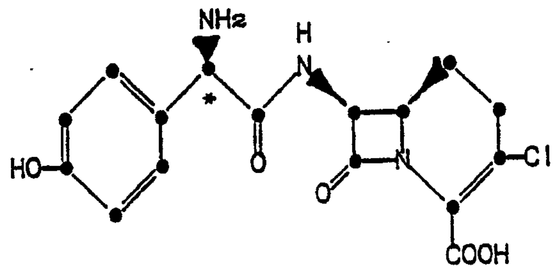

I

where the asterisk indicates R- absolute stereochemistry, is a potent new orally-active antibiotic. The antibiotic is described, for example, in U.S. Patent No. 4,335,211, For brevity's sake, the anhydrous form of the above compound will be referred by the Lilly serial number LY213735.

The present invention is directed to the crystalline monohydrate and the crystalline mono- and bis-(N,N′-dimethylformamide) solvates of LY213735. The latter two solvates will be referred to hereinafter as the "mono(DMF)" and "bis(DMF)" solvates, respectively.

LY213735 crystalline monohydrate is a pharmaceutically elegant hydrate of LY213735. The monohydrate affords a necessary form of LY213735 useful in the manufacture of the various dosage forms of the antibiotic. The crystalline mono(DMF) and bis(DMF) solvates are convenient intermediates to LY213735 in general and the crystalline monohydrate specifically.

One aspect of the present invention is directed to the crystalline monohydrate of LY213735. The formula for LY213735 is given above as Formula I. More specifically, the invention is directed to LY213735 crystalline monohydrate having the x-ray powder diffraction pattern listed below in Table 1. A related aspect are pharmaceutical formulations of LY213735 crystalline monohydrate, of which pharmaceutical formulations containing crystalline LY213735 monohydrate having the x-ray pattern listed in Table 1 are preferred.

Another aspect of the invention is the crystalline mono(DMF) solvate of LY213735. A preferred form of the mono(DMF) solvate is a crystalline compound having the x-ray powder diffraction pattern listed below in Table 2.

Yet another aspect of the instant invention is the crystalline bis(DMF) solvate of LY213735. A preferred form of the bis(DMF) solvate is a crystalline compound having the x-ray powder diffraction pattern listed below in Table 3.

The crystalline mono- and bis(DMF) solvates are convenient intermediates to the corresponding crystalline monohydrate of LY213735. The monohydrate affords a stable, easy-to-handle form of LY213735, a compound that heretofore was both difficult to purify and obtain in a pharmaceutically elegant form.

The instant solvates and monohydrate (and pharmaceutical compositions of the monohydrate) encompass the zwitterionic form of the compound of Formula I. The compounds of this invention refer to the crystalline or microcrystalline form of the monohydrate or the two solvates.

Finally, the instant invention also encompasses pharmaceutical compositions of the crystalline LY213735 monohydrate, and preferably of the monohydrate exhibiting the crystal pattern listed below in Table 1.

A preferred embodiment of the invention is a crystalline monohydrate exhibiting the x-ray powder diffraction pattern of Table 1:

Table 1

| Monohydrate | |
|---|---|
| d | $I/I_1$ |
| 12.99 | .17 |
| 10.76 | 1.00 |
| 9.11 | .33 |
| 7.43 | .14 |
| 6.65 | .12 |
| 5.75 | .05 |
| 5.24 | .04 |
| 5.06 | .16 |
| 4.87 | .24 |
| 4.74 | .23 |
| 4.41 | .23 |
| 4.30 | .18 |
| 4.13 | .12 |
| 3.90 | .27 |
| 3.59 | .22 |
| 3.37 | .53 |
| 3.20 | .12 |
| 2.94 | .10 |
| 2.86 | .06 |
| 2.73 | .06 |

The diffraction pattern in Table 1 was obtained with nickel-filtered radiation of wavelength $\lambda$ = 1.5406 Å. The interplanar spacings are in the column marked "d" and the relative intensities are in the column marked "$I/I_1$".

Another preferred embodiment of the instant invention is the crystalline mono(DMF) solvate exhibiting the x-ray powder diffraction pattern set forth below in Table 2:

Table 2

| Mono(DMF) Solvate | |
|---|---|
| d | $I/I_1$ |
| 13.18 | .12 |
| 10.90 | 1.00 |
| 7.69 | .11 |
| 6.80 | .07 |
| 6.02 | .20 |
| 5.37 | .07 |
| 5.12 | .10 |
| 4.90 | .07 |
| 4.69 | .27 |
| 4.37 | .43 |
| 3.93 | .25 |
| 3.57 | .15 |
| 3.40 | .23 |
| 3.22 | .10 |
| 3.08 | .05 |
| 2.85 | .07 |
| 2.74 | .15 |

The x-ray data in Table 2 was collected with the same instrument parameters used to collect the data in Table 1.

Still another preferred embodiment of the invention is a crystalline form of the bis(DMF) solvate exhibiting the x-ray powder diffraction data listed in the following Table 3:

Table 3

| Bis(DMF) Solvate | |
|---|---|
| d | $I/I_1$ |
| 20.1 | .03 |
| 12.44 | 1.00 |
| 10.27 | .07 |
| 8.58 | .08 |
| 6.91 | .03 |
| 6.60 | .05 |
| 6.23 | .08 |
| 5.43 | .13 |
| 4.74 | .09 |
| 4.41 | .09 |
| 4.19 | .40 |
| 3.62 | .05 |
| 3.52 | .05 |
| 3.39 | .03 |
| 3.34 | .03 |
| 3.14 | .10 |
| 3.02 | .07 |
| 2.86 | .06 |

The x-ray data in Table 3 was collected with the same instrument parameters as those used to collect the data in Table 1.

A further aspect of this invention is a pharmaceutical formulation comprising as an active ingredient a crystalline monohydrate of the compound of Formula (I) associated with one or more pharmaceutically acceptable carriers, vehicles or excipients therefor. In particular, these pharmaceutical compositions are useful for the control of gram-positive and gram-negative bacterial infections in warm-blooded animals and comprise a suitable carrier, vehicle or excipient and a therapeutically effective amount of the LY213735 monohydrate.

With regard to compositions for oral administration (such as tablets and capsules), the term "suitable carrier, vehicle or excipient" means common excipients such as binding agents, for example, syrup, acacia, gelatin, sorbitol, tragacanth, polyvinylpyrrolidine (Povidone), methylcellulose, ethylcellulose, sodium carboxymethylcellulose, hydroxypropylmethylcellulose, sucrose, and starch; fillers and carriers, for example corn starch, gelatin, lactose, sucrose, microcrystalline cellulose, kaolin, mannitol, dicalcium phosphate, sodium chloride, and alginic acid; disintegrators such as croscarmellose sodium, microcrystalline cellulose, corn starch, sodium starch glycolate, alginic acid and mutable wetting agents such as sodium lauryl sulfate; and lubricants such as magnesium stearate and other metallic stearates, stearic acid, silicone fluid, talc, waxes, oils and colloidal silica. Flavoring agents such as peppermint, oil of wintergreen, cherry flavoring or the like can also be used. It may be desirable to add a coloring agent to make the dosage form more aesthetically pleasing in appearance or to help identify the product. The tablets may also be coated by methods well known in the art.

The pharmaceutical compositions of the present invention may also be in the form of oral liquid preparations, which may be either a) aqueous or oily suspensions, solutions, emulsions or syrups; or b) a dry powder to be reconstituted with water or another suitable vehicle before use. When used in conjunction with such oral liquid preparations, the term "suitable carrier, vehicle or excipient" means conventional additives such as suspending agents such as acacia, methyl cellulose, hydroxyethyl cellulose, carboxymethyl cellulose, cellulose with sodium carboxymethyl cellulose (Avicel®), xantham gum, or starch; sweeteners such as sucrose, syrup, glucose, saccharin, sorbital, or aspartame; wetting agents such as

sodium lauryl sulfate, silicone oil, the various Pluromics® surfactants, or glycerin; preservatives such as methyl, propyl, or butyl p-hydroxy benzoates, or sorbic acid; dyes, flavors and salts such as sodium chloride, citric acid, oil of wintergreen or sodium citrate; and waters, oils, and esters such as almond oil, fractionated coconut oil, hydrogenated caster oil, lecithin, aluminum stearate, and the like.

The pharmaceutical composition can also be for intravenous (IV) use. Specifically, a water soluble form of the monohydrate compound can be dissolved in one of the commonly used intravenous fluids and administered by infusion. When used in conjunction with compositions for IV use, the term "suitable carrier, vehicle or excipient" means such fluids as physiological saline, Ringer's solution or 5% dextrose solution.

Topical compositions can be formulated with "suitable carrier, vehicles or excipients" such as hydrophobic or hydrophilic bases. Such bases include ointments, creams or lotions.

Veterinary pharmaceutical compositions of the crystalline monohydrate compound may be administered in the feed or the drinking water of farm animals. Alternatively, the compounds can be formulated as intramammary preparations with "suitable carrier, vehicles or excipients" such as long- or quick-release bases.

The crystalline monohydrate compound of Formula I can also be formulated in unit dosage form in sterile vials, sterile plastic pouches containing a port with a septum, or sterile, hermetically sealed ampoules. The amount of the monohydrate compound per unit dosage may vary from about 100 milligrams to about 10 grams.

A "therapeutically effective amount" of the crystalline monohydrate is from approximately 2.5 mg to about 70 mg of compound per kilogram of body weight per dose. This amount generally totals from about 1 gram to about 5 grams per day for an adult human.

The dose can be administered in a single daily dose or in multiple doses per day. The treatment regimen with the instant formulations may require administration over extended periods of time, for example, for several days or for from two to three weeks. The amount administered per dose or the total amount administered will depend on such factors as the nature and severity of the infection, the age and general health of the patient, and the tolerance to the monohydrate compound of both the patient and the microorganism or microorganisms involved in the infection.

A preferred group of pharmaceutical formulations are formulations comprising a suitable carrier, vehicle or excipient and the crystalline monohydrate exhibiting the x-ray pattern of Table 1, above.

This invention also provides a process for preparing the crystalline monohydrate of the compound of Formula (I) which comprises adjusting the pH of an aqueous solution containing a compound of Formula (I) to about 2 to 7.

The crystalline monohydrate can be made from either the mono(DMF) or bis(DMF) solvate. For instance, the bis(DMF) solvate, along with tetrasodium EDTA and a small amount of concentrated hydrochloric acid, are combined in water. After the suspension forms a solution, the solution is cooled (to approximately 10 °C) and the pH is raised slowly (by the addition of a base such as triethylamine) until a suspension forms (preferably, pH 3.6). The suspended solid (which is the monohydrate) is collected by filtration, washed and dried in the usual manner. Alternatively, the mono(DMF) compound can be converted to the monohydrate under conditions similar to those for the conversion of the bis(DMF) solvate. Upon addition of the base, the pH of the mono(DMF) solution preferably goes to a higher pH than the pH of the bis(DMF) (for example, a pH of 5.8) for a similar amount of base.

This invention also provides a process for preparing the crystalline bis-(N,N′-dimethylformamide) solvate of the compound of Formula (I) which comprises reacting a 7β-amino compound of the Formula (II)

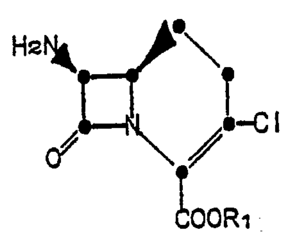

(II)

where $R_1$ is a carboxy-protecting group with an acylating agent of the formula

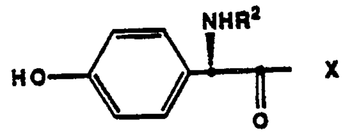

where X is a leaving group and $R^2$ is a protecting group, in DMF followed by deprotection.

The bis(DMF) solvate may result from the acylation of a 7β-amino ("nucleus") compound of the Formula (II)

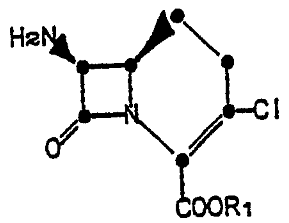

(II)

then removing the carboxy-protecting group represented above as $R_1$. The nucleus and its synthesis are disclosed in U.S. Patent No. 4,734,494.

The carboxy-protecting group $R_1$ of Formula II is a conventional carboxy-protecting group and preferably one which is not sterically hindered. Examples of such groups are the benzyl group and substituted benzyl groups such as 4-methoxybenzyl, 4-nitrobenzyl, 4-methylbenzyl, 3,5-dimethylbenzyl, and 4-chlorobenzyl; silyl groups such as a trialkylsilyl group (in particular, trimethylsilyl); and halo-substituted alkyl groups such as the 2,2,2-trichloroethyl, 2,2,2-tribromoethyl, and 2-iodoethyl groups. A preferred ester group is the benzyl or a substituted benzyl ester group (such as the p-nitrobenzyl group).

In particular, the acylation of the p-nitrobenzyl nucleus compound ($R_1$ = para-nitrobenzyl takes place in cooled (for example, -20° C) DMF. The acylating agent, an activated derivative of 2-(R)-2-(p-hydroxyphenyl)-2-aminoacetic acid, is added to the cooled DMF. A preferred acylating agent is a compound of the Formula (III)

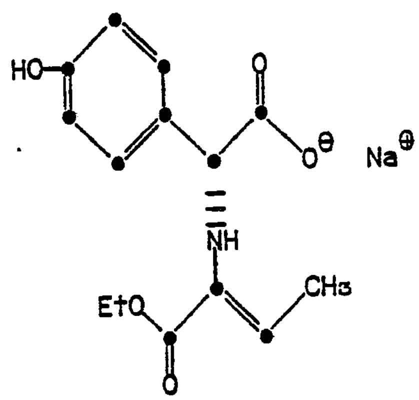

(III)

The reaction solution is cooled, and when III is the acylating agent, methanesulfonic acid, dimethylbenzylamine, and methyl chloroformate are added in rapid succession. The solution is stirred and maintained at a very low (approximately -50° C) temperature, then the pNB ester of the nucleus is added with stirring. The reaction is stirred at low temperature (for example, -45° C) until the acylation reaction is substantially complete (as determined by conventional means such as thin layer chromatography). The mixture is then warmed slowly approximately -10° C and the reagents for removing the amino and carboxy protecting groups (such as water, concentrated hydrochloric acid, and zinc dust for the pNB ester) are added slowly while maintaining the initial temperature of the solution. The solution is stirred at room temperature until the reaction is complete. In the case where $R_1$ was the pNB ester the pH is raised (for example to 2.9) by the addition of a base such as triethylamine, and the resultant zinc residue is removed by filtration. The pH is gradually taken higher until a white suspension formed and the pH remained stable without the addition of base (typically around pH 5.6). (The mixture is seeded with LY213735 at about pH 4.6 to induce

crystallization). The solid phase of the suspension is collected by filtration. The wet filter cake is suspended in a 90:10 mixture of 9:1 DMF/$H_2O$ and solution effected with concentrated hydrochloric acid. The solution is cooled and the pH raised in small increments with a base (triethylamine) until a suspension formed and the pH of the solution stabilized with further additions of base (for example, a pH of approximately 5.7). The crystals are again collected by filtration and dried to give the bis(DMF) solvate.

Alternatively, the bis(DMF) solvate can be made from a concentrated DMF solution of mono(DMF) solvate. Specifically, anti-solvent (preferably acetonitrile) is added in equal volume to the concentrated DMF solution and the mixture is cooled (for example, to 0° C). The solid bis(DMF) precipitate is collected by filtration as above. This invention also provides a process for preparing the crystalline bis(N,N′-dimethylformamide) solvate of the compound of Formula (I) which comprises adding an anti-solvent to a concentrated solution of DMF containing the mono(DMF) solvate of the compound of Formula (I).

The mono(DMF) solvate often results from the acylation reaction used for making the bis(DMF) solvate. Therefore, this invention also provides a process for preparing the crystalline mono(N,N′-dimethylformamide) solvate of the compound of Formula (I) which comprises reacting a 7β-amino compound of the Formula (II)

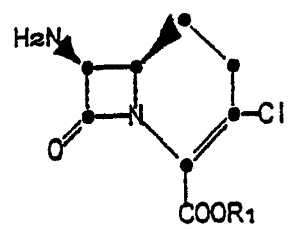

(II)

where $R_1$ is a carboxy-protecting group with an acylating agent of the formula

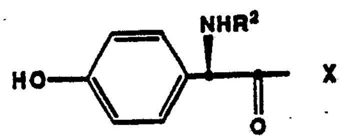

where X is a leaving group and $R_2$ is a protecting group, in DMF followed by deprotection.

Another way of making the mono(DMF) solvate entails suspending the bis(DMF) solvate in a minimum amount of cool 9:1 DMF:$H_2O$ then effecting solution with the minimum amount of acid (such as concentrated hydrochloric acid). The pH of the solution is slowly raised with base (such as triethylamine) until a light suspension forms (typically around pH 3.0). The suspension is filtered (which removes triethylammonium chloride) then the pH of the filtrate is slowly raised to about 5 to 6, preferably 5.8) by the addition of base. The (DMF) crystals are collected by filtration and dried. As such, this invention also provides a process for preparing the mono(N,N′-dimethylformamide) solvate of the compound of Formula (I) as defined above which comprises adjusting the pH of a solution of the bis(DMF) solvate of the compound of Formula (I) in DMF:water (9:1, v:v) to about 5 to 6.

Many alternate methods exist for the acylation of the nucleus compound (Formula II) to give LY213735. LY213735 obtained from these alternate methods could be converted to either one of the two instant DMF solvates by suspending the acylated product compound in a water/DMF mixture, then effecting solution with acid and inducing precipitation with base, as described above. The acylation methods for LY213735 are similar to the methods for the acylation of 6-aminopenicillanic acid, 7-aminodesacetoxycephalosporanic acid, and 7-aminocephalosporanic acid. One acylation method is to simply combine the 7β-amino nucleus with an acid chloride or acid bromide in the presence of an acid scavenger. The acid chloride or acid bromide may be formed in situ. Another method is to combine the 7β-amino nucleus with the free carboxylic acid form of the side chain (or its acid salt) and a condensing agent. Suitable condensing agents include N,N′-disubstituted carbodiimides such as N,N′-dicyclohexylcarbodiimide, N,N′-diethylcarbodiimide, N,N′-di(n-propyl)carbodiimide, N,N′-di(iso-propyl)carbodiimide, N,N′-diallylcarbodiimide, N,N′-bis(p-dimethylaminophenyl)carbodiimide, N-ethyl N′-(4″-ethylmorpholinyl)carbodiimide, and the like. Other suitable carbodiimide condensing agents are disclosed by Sheehan in U.S. Patent No. 2,938,892, and by Hofmann et al. in U.S. Patent No. 3,065,224. Azolides, such as N,N′-carbonyldiimidazole and N,N′-thionyldiimidazole, may also be used as condensing agents. Dehydrating agents such as phosphorus

oxychloride, the alkoxyacetylenes, and 2-halogenopyridinium salts (such as 2-chloropyridinium methyl iodide, 2-fluoropyridinium methyl iodide, and the like) may be used to couple the free acid or its acid salt with the 7β-amino nucleus.

Another acylation method entails first converting the free carboxylic acid form (or the corresponding salt) of the acyl side chain to the corresponding active ester derivative, which is in turn used to acylate the nucleus. The active ester derivative is formed by esterifying the free acid form with groups such as p-nitrophenol, 2,4-dinitrophenol, trichlorophenol, pentachlorophenol, 2-chloro-4,6-dimethoxytriazene, N-chlorosuccinimide, N-chloro maleic imide, N-chlorophthalimide, 1-hydroxy-1H-benzotriazole or 1-hydroxy-6-chloro-1H-benzotriazole. The active ester derivatives can also be mixed anhydrides, which are formed with groups such as methoxycarbonyl, ethoxycarbonyl, iso-butoxycarbonyl, trichloromethylcarbonyl, and iso-but-2-ylcarbonyl, and the carboxylic acid of the acyl side chain. The mixed anhydrides are synthesized by acylating the carboxylic acid of the acyl side chain.

Alternatively, the 7β-amino nucleus can be acylated with the N-ethoxycarbonyl-2-ethoxy-1,2-dihydroquinoline (EEDQ) derivative of the acyl side chain. In general, the free acid form of the acyl side chain and EEDQ are reacted in an inert, polar organic solvent (such as tetrahydrofuran, acetonitrile, and the like). The resultant EEDQ derivative is used in situ to acylate the 7β-amino nucleus.

Yet another method of acylating the 7β-amino compounds entails the use of an enzymatically-assisted process. Such a process is described in Hashimoto et al., U.S. Patent No. 4,335,211, issued June 15, 1982, herein incorporated by reference.

The amino- and carboxy-protecting groups are removed by methods well known in the art. Examples of conditions for the removal of these two types of protecting groups can be found in standard works on the subject such as E. Haslam, "Protective Groups in Organic Chemistry", J.G.W. McOmie, Ed., Plenum Press, New York, N.Y., 1973, Chapters 2 and 5, and T.W. Greene, "Protective Groups in Organic Synthesis", John Wiley and Sons, New York, N.Y., 1981, Chapters 5 and 7, respectively.

Examples of procedures for the removal of amino- and carboxy-protecting groups can also be found in the Experimental Section. For example, the t-butoxycarbonyl amino-protecting group was removed with trifluoroacetic acid, and the p-nitrobenzyl carboxyprotecting group was removed by hydrogenolysis.

In the following Preparations and Examples, the terms dimethylformamide, nuclear magnetic resonance spectra, are abbreviated DMF and n.m.r., respectively.

In conjunction with the n.m.r. spectra, the following abbreviations are used: "s" is singlet, "d" is doublet, "dd" is doublet of doublets, "t" is triplet, "q" is quartet, and "m" is multiplet, respectively.

The n.m.r. spectra were obtained on a General Electric QE-300 300 MHz instrument. The chemical shifts are expressed in δ values (parts per million downfield from tetramethylsilane).

## Experimental Section

### Example 1

LY213735 Bis(DMF) solvate

#### A. Acylation

DMF (81.2 ml) was cooled to -20°C then sodium 2-(R)-2-(p-hydroxyphenyl)-2-(((Z)-methyl but-2-en-2-yloate)amino)acetate (5.70 g, 19.84 mmol) was added to give a white suspension. The suspension was cooled to -45°C. (The pH of the solution was approximately 7.5). Methanesulfonic acid (17.1%, 3.08 mmol, 0.2 ml) was added to the suspension followed immediately by the addition of dimethylbenzylamine (2%, 0.054 ml, 0.37 mmol) and methyl chloroformate (1.42 ml, 18.4 mmol), all the while maintaining the temperature of the suspension below -45°C. The suspension was stirred for an additional 50 minutes at a temperature that fluctuated between -50° and -45°C. p-Nitrobenzyl 7-(R)-7-amino-3-chloro-3-(1-carbadethiacephem)-4-carboxylate (6.33 g, 18.4 mmol, dissovled in DMF (20 ml)) was added to the suspension

while maintaining the temperature of the suspension below -45° C. The suspension was stirred for an additional 1.5 hours then warmed over a 10 minute period to 0° C to give the acylated product.

B. Deprotection

To the solution from A ("Acylation") above, water (6.48 ml), concentrated hydrochloric acid (11.6 ml), and zinc dust (3.34 g, 51.05 mmol, 2.83 equivalents) were added while maintaining the mixture from between 0 to 10° C. The reaction mixture was stirred at room temperature for approximately 5 hours yielding a solution with a red-orange color and a pH of 3.8. Additional concentrated hydrochloric acid (8.9 ml) was introduced and the reaction mixture was stirred for an additional 15 minutes at approximately 20° C. The reaction mixture was cooled to 15° C and the pH of the mixture was raised from 0.99 to 2.9 by the addition of triethylamine (approximately 14 ml). The mixture was stirred for 15 mintues then filtered through Whatman papers and glass filters which were washed with DMF (10 ml). The filtrate was placed in a clean flask, cooled to 15° C, and the pH of the solution was taken to 4.6 by the addition of triethylamine. The solution was seeded with crystals of LY213735. The seeded solution was stirred for 30 minutes then the pH was taken to 5.6 by the addition of triethylamine. The developing mixture was stirred for 35 minutes then filtered. The collected solid was dried in vacuo at room temperature for 3 days to give LY213735 bis(DMF) solvate. (2.99 g, 32.4% yield).

The dried LY213735 bis(DMF) was suspended in 9:1 DMF:water (30 ml) and the pH of the suspension was taken to 1.1 by the addition of concentrated hydrochloric acid. The suspension was stirred for approximately 10 minutes until a solution formed. The pH of the solution was slowly raised to 5.6 by the addition of triethylamine while maintaining the temperature of the solution at 5° C. Upon reaching pH 5.6 the mixture was stirred for 30 minutes and filtered. The collected solid was dried in vacuum at room temperature for 24 hours to give crystalline LY213735 bis(DMF) solvate. n.m.r.: (300 MHz, $D_2O$/DCl) δ: 8.1 (s, 7H) (excess of DMF); 7.6 (2H, d); 7.1 (2H, d); 6.5 (1H, d); 6.3 (1H, s); 4.0 (1H, m); 3.1 (25H(DMF), s); 2.9 (27H(DMF), s); 2.6 (2H, m).

Example 2

LY213735 mono(DMF) solvate

A. Acylation

Under a nitrogen atmosphere, sodium 2-(R)-2-(p-hydroxyphenyl)-2-(((Z)-methyl but-2-en-2-yloate)-amino)acetate (40.57 g, 141.2 mmol) was suspended in DMF (578 ml) that had been cooled to -20° C. The suspension was further cooled to -45° C and methanesulfonic acid (0.33 ml, 5.14 mmol), dimethylbenzyl amine (0.41 ml, 2.69 mmol), and methyl chloroformate (10.62 ml, 137.4 mmol) were added with a syringe. During the addition the temperature of the DMF solution was maintained in a range between -45° to -50° C. The resultant suspension was stirred for an additional 15 minutes at -50° C. p-Nitrobenzyl 7-(R)-7-amino-3-chloro-3-(1-carbadethiacephem)-4-carboxylate (128.4 mmol in DMF (in 190 ml)) was added over a 50 minute period, and the resultant reaction mixture was then stirred at -45° C for 2 hours. The reaction mixture was warmed to -10° C over a 20 minute period to give a solution of the acylated, carboxy-protected product.

B. Deprotection and Crystallization

To the solution from paragraph A above, water (48.8 ml), concentrated hydrochloric acid (87.3 ml), and zinc dust (25.18 g, 385.2 mmol) were added over a 15 minute period while maintaining the temperature of

the reaction mixture between 0 to about 10° C. A final portion of concentration hydrochloric acid (63.3 ml) was added to the solution and the solution was stirred at room temperature for 4.75 hours. The solution was cooled to 15° C, the pH was adjusted to 2.9 by the addition of triethylamine (approximately 100 ml), and the mixture was stirred for an additional 15 minutes. The mixture was filtered to recover the zinc residue then the filtrate was cooled to 15° C and the pH of the filtrate was raised to 4.6 by the addition of triethylamine. The mixture was seeded with the non-solvated form of LY213735 and stirred for 15 minutes. The growing suspension was slowly treated with triethylamine (1 drop every 3 seconds) to obtain a pH of 5.65. The resultant suspension was stirred for several minutes. Additional triethylamine was added to raise the pH of the suspension to 5.866. The resultant thick suspension was stirred for 20 minutes during which time the pH stabilized at 5.833. The suspension was filtered through No. 1 Whatman Paper, and the collected solid was dried in vacuo overnight at room temperature to give a wet mass of 46.49 g.

The wet cake was suspended in 9:1 DMF:water (425 ml) and the pH of the suspension was adjusted to 1.1 with concentrated hydrochloric acid (14 ml), resulting in a solution. After the solution had formed and the pH of the solution had stabilized, the solution was filtered through No. 1 Whatman Paper. The filtrate was cooled to 5° C and the pH was raised to 5.2 with the addition of triethylamine. The mixture was stirred for approximately 30 minutes starting from a time when a suspension first started to form. During those thirty minutes the pH of the mixture dropped to 4.4 and then was taken back to 5.7 with triethylamine (a total of 22 ml). The suspension was stirred until the pH was relatively stable then filtered. The collected solid was washed with a 9:1 DMF:water (20 ml). The washed solid was dried in vacuo at room temperature under a nitrogen flow overnight to yield a mass of 1.83 g, 27.9% of the crystalline LY213735 mono(DMF) solvate. n.m.r.: (300 MHz, $D_2O$/DCl) δ: 7.94 (1H, s, (DMF)); 7.24 (2H, d); 6.94 (2H, d); 5.14 (2H, d); 3.94 (1H, m); 2.97 (3H, s(DMF)); 2.89 (3H, s(DMF)); 2.50 (2H, m); and 2.61 (1H, m).

## Example 3

### LY213735 Monohydrate from the Bis(DMF) Solvate

Water (16.4 ml), and a solution of tetrasodium ethylenediaminetetraacetate (EDTA) (0.03 g) in concentrated hydrochloric acid (0.8 ml) were combined and then LY213735 bis(DMF) solvate (3.0 g) was added. The resultant suspension was stirred until a solution was effected. Charcoal (Darco®, 0.03 g) was added and the suspension was stirred for 10 minutes at 15° C and then filtered through a HYFLO® filter aid pad. The filter cake was washed with water (3 ml). The filtrate (plus the wash) was cooled to 10° C. The pH of the cooled filtrate was taken to 3.6 by the addition of triethylamine to give a thick white suspension. The suspension was stirred for 2 hours and filtered. The filter cake was washed with cold water then dried in vacuo at room temperature overnight to give 1.05 g of crystalline LY213735 monohydrate: Karl Fischer: 3.13% water; n.m.r.: (300 MHz, $D_2O$/DCl) δ: 7.03 (2H, d); 6.6 (2H, d); 5.0 (1H, d); 4.95 (1H, s); 3.6 (1H, m); 2.1 (2H, m); 1.0 (2H, m); 0.94 (1H, t); X-ray: (The powder X-ray diffraction parameters and pattern are given above as Table 1). Analysis Calculated for $C_{16}H_{18}N_3O_6Cl$:
Theory: C, 50.07; H, 4.73; N, 10.95;
Found: C, 50.20; H, 5.02; N, 11.17.

## Example 4

### LY213735 Mono(DMF) Solvate from the Corresponding Bis(DMF) Solvate

A mixture of the mono(N′,N-dimethylformamide) solvate and bis(N,N′-dimethylformamide) solvate compounds (a total of 70.64 g) was combined in the minimum amount of DMF (250 ml). The suspension was cooled to between 10 and 15° C. The pH of the cooled suspension was adjusted to 1.1 by the addition of concentrated hydrochloric acid. The acidified suspension was stirred until a solution was obtained. The pH of the solution was slowly adjusted to 5.8 with triethylamine, all the while stirring the solution very slowly

(approximately 1 revolution per second). The resultant suspension was stirred slowly for 15 minutes while the pH was maintained between 5.7 and 5.8 then filtered. The filter cake washed with ethyl acetate and dried in vacuo overnight to yield 47.3 g of crystalline LY213735 mono(DMF) solvate. Karl Fischer: 8.72% water; n.m.r.:(300 MHz, $D_2O/DCl$) δ: 7.9 ($\frac{1}{2}$ H, s(DMF)); 7.4 (2H, d); 6.75 (2H, d); 5.45 (1H, d); 4.6 (1H, s); 3.75 (1H, m); 2.75 (2H, s(DMF)); 2.4 (2H, s(DMF)); 2.55 (2H, m); 1.45 (2H, m); X-ray: (The powder diffraction X-ray parameters and pattern for this compound are given above in conjunction with Table 2).

## Example 5

LY213735 Monohydrate from the Corresponding Mono(DMF) Solvate

Deionized water (300 ml), tetrasodium ethylenediaminetetraacetate (EDTA) (0.52 g, 1.2 mmol), and concentrated hydrochloric acid (14.2 ml) were combined and stirred first at room temperature then cooled to 15° C. LY213735 mono(DMF) solvate (47.0 g, 0.107 mole) was added to the solution and the resultant suspension became a solution after approximate 10 mintues of stirring. Charcoal (Darco®, 0.5 g) was added and then the suspension was stirred for 10 minutes at a temperature between about 15° to 20° C. The suspension was filtered through an 0.5 inch pad of HYFLO® filter aid. The filter cake washed with water (approximately 60 ml). The (combined) filtrate (and wash) was cooled to 15° C and the pH was raised slowly (0.1 units per 5 minutes) to 1.7 by the addition of triethylamine. The cloudy solution was stirred for approximately 10 minutes to give a thick white suspension. The suspension was stirred for another 45 mintues. The pH of the suspension was then slowly raised to 3.5 by the addition of triethylamine. The pH of the suspension continued to rise spontaneously to 5.8 where upon several drops of concentrated hydrochloric acid were added to steady the pH at 5.75. The suspension was stirred for 1.5 hours at this pH 5.75 while maintaining the temperature of the suspension from between about 13 to about 16° C, then filtered. The filter cake was washed with water (60 ml) and was dried in vacuo at room temperature for overnight to give 36.06 g of a white solid.

The filtrate from the final filtration of the above paragraph was diluted with acetonitrile (400 ml), and chilled overnight to yield a white suspension. The suspension was filtered and dried in vacuo overnight to yield 2.48 g of a white solid.

The two lots of white solids (from the first and second paragraphs above) were combined in water (200 ml). The resultant suspension was stirred at approximately 10° C for 1 hour and was filtered. The filter cake was washed with water (approximately 50 ml) was dried in vacuo overnight at room temperature to yield 28.39 g of crystalline LY213735 monohydrate: Karl Fischer analysis: 2.6% water; n.m.r.: (300 MHz, $D_2O/DCl$) δ: 7.20 (2H, d); 6.75 (2H, d); 5.1 (1H, d); 5.0 (1H, s); 3.7 (1H, m); 2.7 ($\frac{1}{3}$ H, s); 2.35 (2H, m); 1.05 (2H, m).

## Example 6

Hard gelatin capsules are prepared using the following ingredients:

| | Quantity (mg/capsule) |
|---|---|
| LY213735 crystalline monohydrate | 200 |
| Starch flowable powder | 200 |
| Starch Flowable pattern with silicone 5% | 50 |
| Magnesium stearate | 2.5 |

The above ingredients are mixed and filled into hard gelatin capsules in 452.5 mg quantities.

## Example 7

A tablet formula is prepared using the ingredients below:

| | Quantity (mg/tablet) |
|---|---|
| LY213735 crystalline monohydrate | 200 |
| Cellulose, microcrystalline | 200 |
| Silicon dioxide, fumed | 10 |
| Stearic acid | 5 |

The components are blended and compressed to form tablets each weighing 415 mg.

## Example 8

Suppositories each containing 200 mg of active ingredient are made as follows:

| | |
|---|---|
| LY213735 crystalline monohydrate | 200 mg |
| Saturated fatty acid glycerides to | 2000 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

## Example 9

| Ready To-Use Aqueous Suspension 200 mg/5 ml | |
|---|---|
| LY213735 crystalline monohydrate | 210 mg |
| Cellulose with sodium carboxymethylcellulose | 95 mg |
| sucrose | 1.85 g |
| parafens | 3 mg |
| emulsion silicone | 2.5 mg |
| pluronic | 5 mg |
| flavor | 2 mg |
| color | 0.5 mg |
| purified water | q.s. to 5 ml |

The ingredients are sieved. The parafens are dissolved in hot purified water and, after cooling, the other ingredients are added. Sufficient purified water is added to produce the required volume. The suspension can then be passed through a colloid mill or homogenizer to produce a more elegant dispersion.

## Example 10

| Suspension for Reconstitution | |
|---|---|
| LY213735 crystalline monohydrate | 210 mg |
| Sucrose | 3 g |
| Xanthan gum | 5 mg |
| starch modified | 10 mg |
| emulsion silicone | 5 mg |
| sodium lauryl sulfate | 0.5 mg |
| methylcellulose | 2 mg |
| flavor | 0.5 mg |
| dye | |

The ingredients are sieved and mixed in a suitable blender, such as a twin-shell, twin core Nauta®-type, or ribbon blender. To constitute, a sufficient volume of purified water is added to produce the required volume.

## Claims

1. A crystalline monohydrate of the compound of the Formula (I)

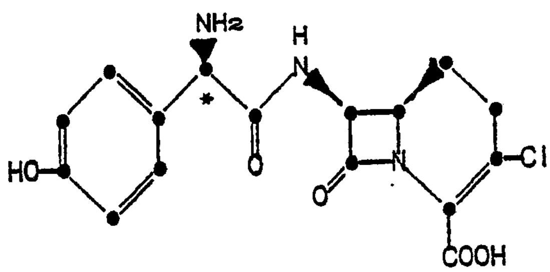

(I)

2. A crystalline mono(N,N′-dimethylformamide) solvate of the compound of the Formula (I)

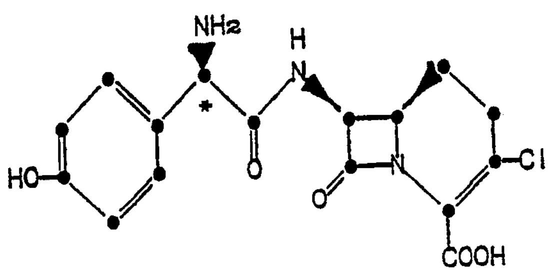

(I)

3. A crystalline bis(N,N′-dimethylformamide) solvate compound of the Formula (I)

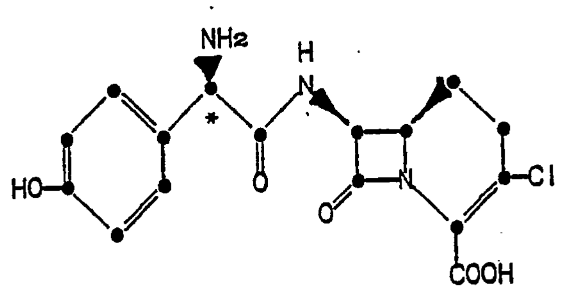

(I)

4. A pharmaceutical formulation comprising as an active ingredient a crystalline monohydrate of the compound of Formula (I) as claimed in Claim 1, associated with one or more pharmaceutically acceptable carriers, vehicles or excipients therefor.

5. A crystalline monohydrate of the compound of Formula (I) as claimed in Claim 1 for use in treating bacterial infections in warm-blooded animals.

6. A process for preparing the crystalline monohydrate of the compound of Formula (I), as claimed in Claim 1, which comprises adjusting the pH of an aqueous solution containing a compound of Formula I to about 2 to 7.

7. A process of Claim 6 wherein the pH is adjusted to about 3.6.

8. A process of Claim 6 wherein the pH is adjusted to about 5.8.

9. A process for preparing the crystalline mono(N,N′-dimethylformamide) solvate of the compound of Formula (I) as claimed in Claim 2 which comprises reacting a 7β-amino compound of the Formula (II)

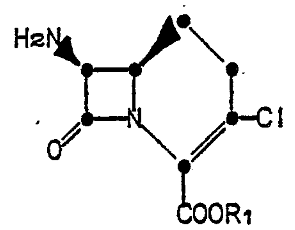

(II)

where $R^1$ is a carboxy-protecting group with an acylating agent of the formula

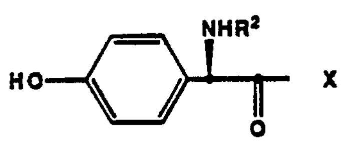

where X is a leaving group and $R^2$ is a protecting group, in DMF followed by deprotection.

10. A process for preparing the mono(N,N′-dimethylformamide) solvate of the compound of Formula (I) as claimed in Claim 2 which comprises adjusting the pH of a solution of the bis(DMF) solvate of the compound of Formula (I) as claimed in Claim 3 in DMF:water (9:1, v:v) to about 5 to 6.

11. A process for preparing the crystalline bis(N,N′-dimethylformamide) solvate of the compound of Formula (I) as claimed in Claim 3 which comprises reacting a 7β-amino compound of the Formula (II)

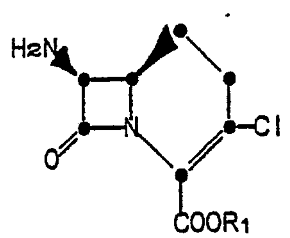

(II)

where $R^1$ is a carboxy-protecting group with an acylating agent of the formula

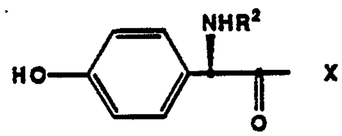

where X is a leaving group and $R^2$ is a protecting group, in DMF followed by deprotection.

12. A process for preparing the crystalline bis(N,N′-dimethylformamide) solvate of the compound of Formula (I) as claimed in Claim 3 which comprises adding an anti-solvent to a concentrated solution of DMF containing the mono(DMF) solvate of the compound of Formula (I) claimed in Claim 2.

Claims for the following Contracting State : GR

1. A process for preparing the crystalline monohydrate of the compound of the Formula (I)

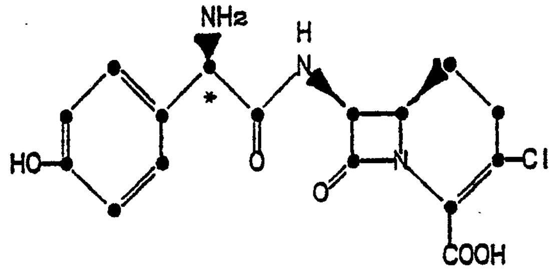

(I)

which comprises adjusting the pH of an aqueous solution containing a compound of Formula (I) to about 2 to 7.

2. A process for preparing the crystalline mono(N,N′-dimethylformamide) solvate of the compound of the Formula (I)

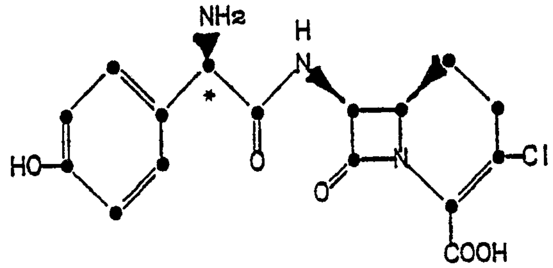

(I)

which comprises reacting a 7β-amino compound of the Formula (II)

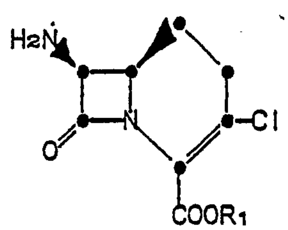

(II)

where $R^1$ is a carboxy-protecting group with an acylating agent of the formula

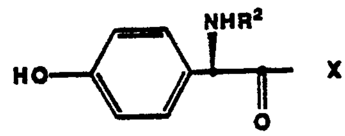

where X is a leaving group and $R^2$ is a protecting group, in DMF followed by deprotection.

3. A process for preparing the mono(N,N′-dimethylformamide) solvate of the compound of Formula (I) as defined in Claim 2 which comprises adjusting the pH of a solution of the bis(DMF) solvate of the compound of Formula (I) as defined in Claim 4 in DMF:water (9:1,v:v) to about 5 to 6.

4. A process for preparing the crystalline bis(N,N′-dimethylformamide) solvate of the compound of the Formula (I)

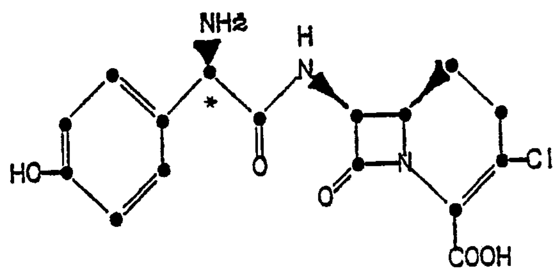

(I)

which comprises reacting a 7β-amino compound of the Formula (II)

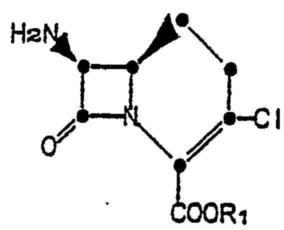

(II)

where $R_1$ is a carboxy-protecting group with an acylating agent of the formula

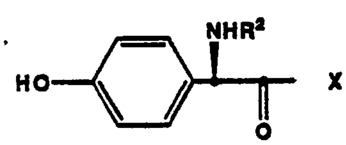

where X is a leaving group and $R^2$ is a protecting group, in DMF followed by deprotection.

5. A process for preparing the crystalline bis(N,N′-dimethylformamide) solvate of the compound of Formula (I) as defined in Claim 4 which comprises adding an anti-solvent to a concentrated solution of DMF containing the mono(DMF) solvate of the compound of Formula (I) as defined in Claim 2.

6. A process for preparing a pharmaceutical formulation comprising admixing a crystalline monohydrate of the compound of Formula (I) as defined in Claim 1 with one or more pharmaceutically acceptable carriers, vehicles or excipients therefor.

7. A crystalline mono(N,N′-dimethylformamide) solvate of the compound of the Formula (I)

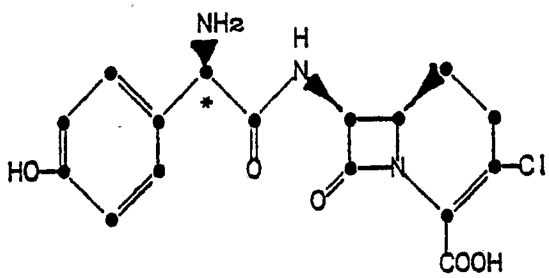

(I)

8. A crystalline bis(N,N′-dimethylformamide) solvate compound of the Formula (I)

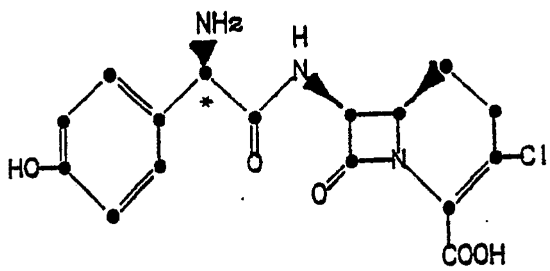

(I)

Claims for the following Contracting State : ES

1. A process for preparing the crystalline monohydrate of the compound of the Formula (I)

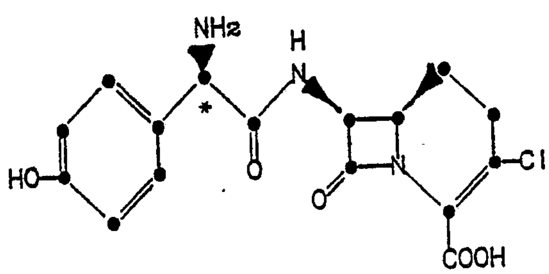

(I)

which comprises adjusting the pH of an aqueous solution containing a compound of Formula (I) to about 2 to 7.

2. A process for preparing the crystalline mono(N,N′-dimethylformamide) solvate of the compound of the Formula (I)

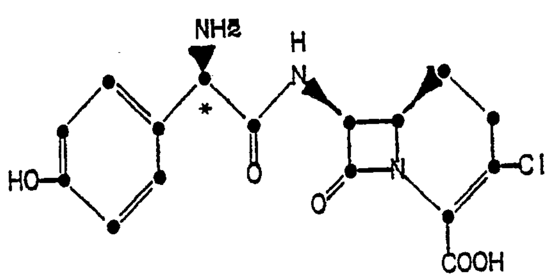

(I)

which comprises reacting a 7β-amino compound of the Formula (II)

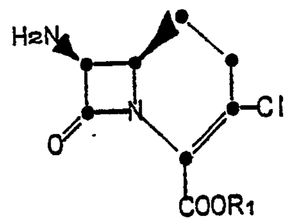

(II)

where $R^1$ is a carboxy-protecting group with an acylating agent of the formula

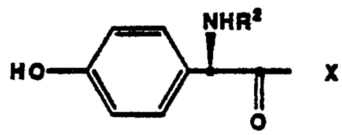

where X is a leaving group and $R^2$ is a protecting group, in DMF followed by deprotection.

3. A process for preparing the mono(N,N′-dimethylformamide) solvate of the compound of Formula (I) as defined in Claim 2 which comprises adjusting the pH of a solution of the bis(DMF) solvate of the compound of Formula (I) as defined in Claim 4 in DMF:water (9:1,v:v) to about 5 to 6.

4. A process for preparing the crystalline bis(N,N′-dimethylformamide) solvate of the compound of the Formula (I)

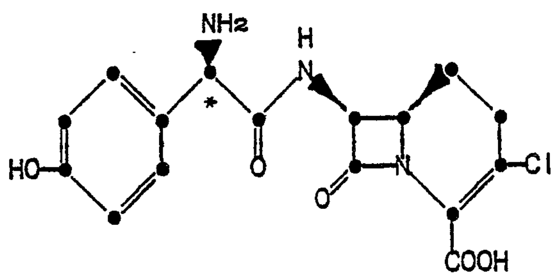

(I)

which comprises reacting a 7β-amino compound of the Formula (II)

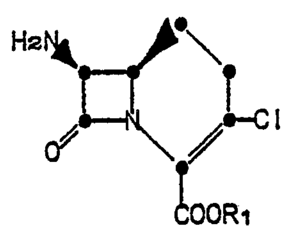

(II)

where $R^1$ is a carboxy-protecting group with an acylating agent of the formula

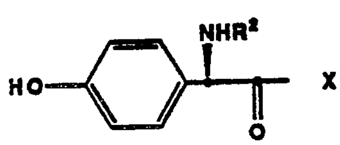

where X is a leaving group and $R^2$ is a protecting group, in DMF followed by deprotection.

5. A process for preparing the crystalline bis(N,N′-dimethylformamide) solvate of the compound of Formula (I) as defined in Claim 4 which comprises adding an anti-solvent to a concentrated solution of DMF containing the mono(DMF) solvate of the compound of Formula (I) as defined in Claim 2.

6. A process for preparing a pharmaceutical formulation comprising admixing a crystalline monohydrate of the compound of Formula (I) as defined in Claim 1 with one or more pharmaceutically acceptable carriers, vehicles or excipients therefor.

European Patent Office

# EUROPEAN SEARCH REPORT

Application Number

EP 88 30 9260

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-4 160 863 (BOUZARD et al.)<br>* Abstract; claims *<br>--- | 1,4 | C 07 D 471/04<br>A 61 K 31/435//<br>(C 07 D 471/04<br>C 07 D 221:00<br>C 07 D 205:00 ) |
| A | US-A-3 957 773 (BURTON et al.)<br>* Whole document *<br>--- | 1-3,6-12 | |
| D,A | US-A-4 335 211 (HASHIMOTO et al.)<br>* Claims; reference example 5 *<br>----- | 1 | |
| | | | **TECHNICAL FIELDS SEARCHED (Int. Cl.4)** |
| | | | C 07 D 471/00<br>C 07 D 501/00<br>A 61 K 31/00 |

The present search report has been drawn up for all claims

| Place of search | Date of completion of the search | Examiner |
|---|---|---|
| THE HAGUE | 06-12-1988 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)